# EUROPEAN PATENT APPLICATION

(11) **EP 4 763 836 A1**
(43) Date of publication of application: **24.06.2026**
(21) Application number: 24221646.3
(22) Date of filing: 19.12.2024
(51) Int. Cl.: C07D 307/50, C07B 63/04

(54) **DISCOLORATION PREVENTION**

(71) Applicant: Stora Enso Oyj, 00101 Helsinki (FI)
(72) Inventor: Torssell, Staffan, 16860 Bromma (SE)
(74) Representative: Brann AB

(57) **Abstract**

The present document is directed to a method for preventing discoloration of a hydrogenated furanic oxidation product comprising furandicarboxylic acid (FDCA), The method comprises adding an antioxidant to a furanic oxidation product comprising FDCA. The antioxidant is added before and/or after hydrogenation of the furanic oxidation product comprising FDCA.

## Description

### TECHNICAL FIELD

The present document is directed to the field of production of furanic oxidation products comprising FDCA. More particularly, the present document is directed to the problem of discoloration of such furanic oxidation products comprising FDCA. In particular, the present document is directed to a method for preventing discoloration of a hydrogenated composition comprising a furanic oxidation product.

### BACKGROUND

The synthesis of bio-based chemicals and polymers from renewable biomass has been developed as a sustainable alternative to reduce dependence on fossil-fuel resources. 2,5-furan dicarboxylic acid (FDCA) can e.g. be used for the production of polyethylene furanoate (PEF), which is a substitute for polyethylene terephthalate (PET). The common route for synthesizing FDCA is by oxidation of 5-hydroxymethylfurfural (5-HMF). Oxidation of 5-HMF to produce FDCA is a complex reaction involving several intermediate molecules such as 5-hydroxymethyl-2-furancarboxylic acid (HMFCA), 2,5-diformylfuran (DFF) and 5-formyl-2-furancarboxylic acid (FFCA).

WO 2017/123763 discloses a process for producing 2,5-furandicarboxylic acid pathway products, such as HMFCA, DFF, FFCA and FDCA. The process involves contacting an oxidation feedstock comprising a furanic oxidation substrate and an oxidation solvent with oxygen in the presence of a heterogenous oxidation catalyst comprising a solid support and a noble metal.

Further, WO 2019/014382 discloses a process for producing a purified 2,5-furandicarboxylic acid pathway product. The process involves contacting an FDCA pathway product comprising FDCA and FFCA with hydrogen in the presence of a heterogenous reduction catalyst and a solvent.

To produce high-purity FDCA crystals from the oxidation product stream of HMF, MMF (5-methoxymethyl furfural) or CMF (5-chloromethyl furfural) or similarly 5-substituted methyl furfurals, on-path oxidation intermediates such as FFCA needs to be removed from the final product. FFCA and other mono-functionalized furan carboxylic acids will act as chain terminators in the subsequent polymerization of FDCA and a diol into polyester and other polymers. FFCA itself, is hard to remove from FDCA since FFCA and FDCA co-crystallize, and low levels are hard to reach. However, if the aldehyde in FFCA is reduced using for example hydrogen and a hydrogenation catalyst to generate HMFCA, high-purity FDCA can be achieved. HMFCA can readily be removed from the FDCA crystals by a recrystallization step in water or a miscible solvent-water blend.

The hydrogenation step is also used to reduce coloured impurities present in the crude stream after the oxidation reaction. The presence of such coloured impurities are not desirable in the purified FDCA as it will lead to discoloration of polymerization products prepared from the FDCA.

Other methods for preventing discoloration of polymerization products involve adding antioxidants to the final, purified FDCA composition or during different steps in the polymerization of FDCA to produce polyethylene furanoate (PEF). For example, US2023192587 discloses either to use hydrogenation to provide a colour stabilized FDCA product or to use antioxidants as colour stabilizing compounds.

However, there still is a need to for efficient method of producing high purity and colorless FDCA.

An object of the present document is thus to overcome or at least mitigate one or more of the problems described herein.

### SUMMARY

An object of the present document is therefore to provide a method for preventing the discoloration of a furanic oxidation product comprising FDCA.

Such as method for preventing discoloration of a hydrogenated furanic oxidation product comprising furandicarboxylic acid (FDCA) comprises adding an antioxidant to a furanic oxidation product comprising FDCA, wherein said antioxidant is added before and/or after hydrogenation of said furanic oxidation product comprising FDCA.

The furanic oxidation product comprising FDCA may further comprise 5-formyl-2-furancarboxylic acid (FFCA) and/or 5-hydroxymethyl-2-furancarboxylic acid (HMFCA).

The furanic oxidation product comprising FDCA may be produced by oxidising a furanic oxidation substrate, such as hydroxymethyl furfural (HMF), diformylfuran (DFF), hydroxymethylfurancarboxylic acid (HMFCA), and 5-formylfurancarboxylic acid (FFCA).

The antioxidant may be a substituted phenol and/or a phosphine. For example, the antioxidant may be ascorbate, ascorbic acid, sodium metabisulfite, butyl hydroxytoluene (BHT), tert-butylhydroquinone (TBHQ) and butylated hydroxyanisole (BHA). A combination of two or more antioxidants may be used.

The method may further comprise keeping said hydrogenated furanic oxidation product comprising FDCA and an antioxidant, at an elevated temperature and/or to a step of re-crystallising the hydrogenated furanic oxidation product comprising FDCA and an antioxidant.

The present document is also directed to a method for producing a colour-stabilized furanic oxidation product comprising FDCA, said method comprising the steps of:
i) providing a furanic oxidation product comprising FDCA;
ii) hydrogenating the furanic oxidation product comprising FDCA of step i) to provide a hydrogenated a furanic oxidation product;
iii) optionally re-crystallising the hydrogenated furanic oxidation product comprising FDCA of step ii),
said method further comprising a step of adding an antioxidant to the furanic oxidation product comprising FDCA of step i) before performing step ii) and/or adding said antioxidant to said hydrogenated furanic oxidation product comprising FDCA of step ii).

The present document is further directed to a composition comprising a hydrogenated a furanic oxidation product comprising FDCA and an antioxidant.

The present document is further directed to a composition comprising a hydrogenated furanic oxidation product comprising FDCA and an antioxidant obtained or obtainable by a method disclosed herein.

The present document also discloses the use of an antioxidant to prevent discoloration of a hydrogenated furanic oxidation product comprising FDCA.

Other features and advantages of the invention will be apparent from the following detailed description, drawings, examples, and from the claims.

### DETAILED DESCRIPTION

As mentioned above, discoloration of FDCA containing compositions is problematic as this may lead to discolored polymerised products, which is not desirable.

However, in the production of compositions comprising a furanic oxidation product comprising FDCA, impurities which may cause discoloration may be formed. Such impurities may also be precursors of colored substances that e.g. may turn into coloured substances during polymerization, causing discoloration of the polymerized product. In particular, such coloured substances or precursors of coloured substances are known to form from reaction intermediates or side products from the oxidation of 5-hydroxymethylfurfural (HMF) during the production of FDCA. One such known impurity which may cause discoloration is 5-formylfuran-2-carboxylic acid (FFCA), an intermediate structure formed during the oxidation of HMF to FDCA.

A furanic oxidation product comprising FDCA may therefore comprise impurities that may cause discoloration of FDCA containing compositions as such, but also of polymerised products prepared from FDCA containing such impurities.

Re-crystallisation of furanic oxidation products comprising FDCA may be used to remove some impurities. For example, HMFCA may be removed by re-crystallisation. However, FFCA is difficult to get rid of using re-crystallisation.

Hydrogenation has also been used to remove impurities that may cause discoloration. In particular, hydrogenation has been used to remove FFCA from furanic oxidation products comprising FDCA. Such hydrogenation may involve contacting the furanic oxidation product comprising FDCA with hydrogen in the presence of a, preferably heterogenous, reduction catalyst and a solvent, such as water or an aqueous solvent. FFCA present in the furanic oxidation product comprising FDCA will in such a reaction be hydrogenated to HMFCA leading to a furanic oxidation product comprising FDCA with a reduced content of FFCA. As mentioned above, if desirable, the content of HMFCA in a furanic oxidation product comprising FDCA may be reduced by re-crystallisation.

The present inventor has now surprisingly found that although hydrogenation may be used to reduce the amount of impurities which may cause discoloration, the hydrogenation in itself can cause problems with discoloration of furanic oxidation products comprising FDCA. This problem is particularly pronounced when a hydrogenated furanic oxidation product comprising FDCA is kept at an elevated temperature after hydrogenation, as is often done if the hydrogenated furanic oxidation product comprising FDCA is to be re-crystallised to remove impurities. Studies on lab scale showed that the slightly coloured crude FDCA solution (i.e. a solution of a furanic oxidation product comprising FDCA) can be hydrogenated using a standard palladium on carbon catalyst to convert the FFCA into HMFCA and small amounts of MFCA (5-methyl furan carboxylic acid). The pale-yellow colour is also removed, and the hydrogenation product is initially a non-coloured transparent solution. However, when the hydrogenation product is stored at elevated temperature, the colourless solution turned brown. Without wishing to be bound by theory, this may be caused by an oxidative process due to dissolved oxygen present in the solvent or entering the solvent from the air reacting with an organic compound present in the solution. Control experiments show that the hydrogenation feed does not gain any colour by the same heat treatment that caused coloration after the hydrogenation step. The colour formation is not caused by anything present in the hydrogenation feed, but it is caused by something formed during the hydrogenation reaction.

The impurities causing discoloration that forms after hydrogenation cannot be completely removed by re-crystallisation, which means that even if the hydrogenated furanic oxidation product comprising FDCA is re-crystallised, at least part of the impurities causing discoloration that formed after hydrogenation remain in the re-crystallised composition. As is demonstrated in the experimental section, the hydrogenated furanic oxidation products comprising FDCA initially has a low degree of discoloration. However, upon storage at an elevated temperature, with time more and more discoloration develop. As also can be seen in the experimental section, this problem seems to be caused by the hydrogenation process itself as a composition comprising a furanic oxidation product comprising FDCA which had not been subjected to hydrogenation does not become discoloured upon storage at an elevated temperature. Apparently, hydrogenation forms products which lead to discoloration, particularly when keeping the hydrogenated furanic oxidation product comprising FDCA at elevated temperatures. This problem has previously not been recognised.

Interestingly, the addition of antioxidants to a furanic oxidation product comprising FDCA before and/or after hydrogenation prevented the discoloration, even when the hydrogenated furanic oxidation product comprising FDCA was kept at elevated temperatures.

Consequently, the present document is directed to a method for preventing discoloration of a hydrogenated furanic oxidation product comprising furandicarboxylic acid (FDCA), wherein the method comprises adding an antioxidant to a furanic oxidation product comprising FDCA, wherein said antioxidant is added before and/or after hydrogenation the furanic oxidation product comprising FDCA.

As mentioned above, the present inventor surprisingly found that although a hydrogenated furanic oxidation product comprising FDCA had a low degree of discoloration immediately after the hydrogenation, a discoloration may appear after hydrogenation, particularly when keeping the hydrogenated furanic oxidation product comprising FDCA at elevated temperatures. The addition of an antioxidant as disclosed herein prevents the development of discolorations or at least reduces the degree to which such discolorations develop. The term "preventing discoloration" and the like as used herein thus refers to preventing and/or reducing the development of discolorations of hydrogenated furanic oxidation products comprising FDCA. Typically, such a prevention of discoloration leads to a hydrogenated furanic oxidation product comprising FDCA, which even after storage has a degree of discoloration which is not more than the discoloration of the furanic oxidation product comprising FDCA before hydrogenation.

The antioxidant may be added to a furanic oxidation product comprising FDCA before hydrogenation and/or after hydrogenation of the furanic oxidation product comprising FDCA (i.e. to the hydrogenated furanic oxidation product). It may be preferred to add the antioxidant after the hydrogenation as the hydrogenation may destroy or impair the antioxidative effect of some antioxidants. The skilled person knows which antioxidants may be sensitive to hydrogenating conditions. The antioxidant should however not be added before oxidation of a furanic oxidation substrate as this may destroy the antioxidant or negatively affect the oxidation reaction. The antioxidant should therefore be added after oxidation of a furanic oxidation substrate to a furanic oxidation product comprising FDCA. When the antioxidant is added after hydrogenation of a furanic oxidation product comprising FDCA, it is preferably added as soon as possible after hydrogenation. In this case, typically, the antioxidant is added to a hydrogenated furanic oxidation product comprising FDCA within 60 minutes after the hydrogenation process is finished. Typically, the antioxidant is added to the furanic oxidation product comprising FDCA and blended into this by mixing. The antioxidant may be in solid or liquid form (concentrated or diluted) when added. Typically, the antioxidant is added as a diluted liquid.

The furanic oxidation product comprises FDCA. Typically, the furanic oxidation product comprises at least from about 2 to about 20 wt% FDCA, such as from about 4 to about 15 wt% FDCA, such as about 2 wt%, 3 wt%, 4 wt%, 5 wt%, 6 wt%, 7 wt%, 8 wt%, 9 wt%, 10 wt%, 11 wt%, 12 wt%, 13 wt%, 14 wt%, or 15 wt% FDCA. Before the hydrogenation, the furanic oxidation product also typically comprises FFCA and/or HMFCA. The amount of FFCA in the furanic oxidation product before hydrogenation is typically 1.5 wt% or less, such as from about 0.1 wt% to about 1.5 wt%. After hydrogenation, the amount of FFCA is typically 0.1 wt% or less. The amount of HMFCA in the furanic oxidation product before hydrogenation is typically 0.1 wt% or less. After hydrogenation, the amount of HMCFA is typically 1.5 wt% or less, such as from about 0.1 wt% to about 1.5 wt% The furanic oxidation product may comprise diformylfuran (DFF) and/or 2-furoic acid. A furanic oxidation product comprising FDCA according to the present document may thus be a composition comprising a furanic oxidation product in the form of FDCA that also comprises other furanic oxidation products such as FFCA, HMFCA, 2-furan acid and/or DFF. Such furanic oxidation products that are not FDCA may in the present document be denoted non-FDCA furans. A furanic oxidation product comprising FDCA as referred to herein may therefore further comprise FFCA, HMFCA, OFF and/or other furanic oxidation products in addition to FDCA. Typically, the furanic oxidation product comprising FDCA and/or a hydrogenated furanic oxidation product comprising FDCA does not contain any terephthalic acid.

If the hydrogenated furanic oxidation product comprising FDCA is to be re-crystallised, the antioxidant is added before the re-crystallisation. When a hydrogenated furanic oxidation product comprising FDCA is to be re-crystallised in order to remove impurities, the composition is typically held at an elevated temperature in order to prevent premature crystallisation. As mentioned above, the problem with discoloration of a hydrogenated furanic oxidation product comprising FDCA is most pronounced when the furanic oxidation product comprising FDCA is held at an elevated temperature, such as for at least 0.5 hours. Further, if a furanic oxidation product is re-crystallised at a high temperature (such as about 170 °C), discoloration may occur. The elevated temperature is typically above the temperature where all compounds in the hydrogenated furanic oxidation product solution are soluble. The furanic oxidation product comprising FDCA is typically held at a temperature of from about 100 °C to about 130 °C, such as from about 105 °C to about 125 °C, such as from about 110 °C to about 120 °C. Keeping the hydrogenated furanic oxidation product comprising FDCA at such a temperature will prevent premature crystallisation. Premature crystallisation may lead to blockages in pipes, valves etc. By premature crystallisation is intended a crystallisation taking place before the crystallization in the intended crystallization vessel.

Any antioxidant may be used as the antioxidant according to the present document. Typically, the antioxidant is a substituted phenol or a phosphine. Substituted phenols are compounds having at least one phenol moiety, but possibly two or more phenol moieties, in which the benzene ring(s) of such moiety or moieties have at least one substituent, other than the hydroxyl substituent. Examples of such substituents are alkoxy and alkyl substituents, such as methoxy and tert-butyl substituents. Therefore, examples of substituted phenols include alkoxy-substituted (e.g., methoxy-substituted) and alkylsubstituted (e.g., tert-butyl-substituted) phenols. Non-limiting examples of substituted phenols include butylated hydroxyanisole (BHA); 2,6-dimethoxyphenol (DMP); 2,6-di-tert-butyl-4-methoxylphenol (DTMP); pentaerythritol tetrakis[3-[3,5-di-tert-butyl-4-hydroxyphenyl]propionate (PETC); 2-tert-butylhydroquinone (TBHQ); ethylenebis (oxyethylene) bis-(3-(5-tert-butyl-4-hydroxy-m-tolyl)-propionate); and octadecyl-3-(3,5-di-tert-butyl-4-hydroxyphenyl)-propionate. The antioxidant may also be a phenyl-substituted amine (e.g., 4,4'-bis(α,α-dimethylbenzyl) diphenylamine (XDPA)), a phosphite (e.g., tris(2,4-di-tert-butylphenyl)phosphite), or an antioxidant vitamin (e.g., ascorbic acid or ascorbate). The antioxidant may be selected from one or more of ascorbate, ascorbic acid, sodium metabisulfite, butyl hydroxy toluene (BHT), tert-butylhydroquinone (TBHQ) and butylated hydroxyanisole (BHA). The antioxidant may be a single antioxidant or a combination of two or more antioxidants may be used. If the antioxidant is added both before and after hydrogenation, the same or different antioxidant(s) or different combinations of antioxidants may be added before and after hydrogenation, in the same or different ratios if a combination of antioxidants is used. The concentration of antioxidant is varied depending on which antioxidant is used in order to achieve the desired prevention of discoloration. This can easily be tested by adding different amounts of antioxidants and see which concentrations result in a satisfactory prevention of discoloration. As mentioned elsewhere herein, typically, such a prevention of discoloration leads to a hydrogenated furanic oxidation product comprising FDCA, which even after storage has a degree of discoloration which is not more than the discoloration of the furanic oxidation product comprising FDCA before hydrogenation. Typically, the amount of antioxidant or total amount of antioxidants if a combination of two or more antioxidants is used is in the range of about 50 ppm to about 1500 ppm, such as from about 50 ppm to about 800 ppm, such about 50 ppm to about 500 ppm, such as from about 50 ppm to about 200 ppm, such as about 150 ppm.

The hydrogenation process used is not critical for the effect of the antioxidant as used according to the present document. An example of a hydrogenation process suitable for use for use in accordance with the present document is disclosed in WO2019014382 which discloses a hydrogenation process comprising contacting a furanic oxidation product comprising FDCA with hydrogen in the presence of a heterogeneous reduction catalyst and a solvent under conditions sufficient to form a reaction mixture for reducing FFCA that may be present in the furanic oxidation product comprising FDCA to hydroxymethylfurancarboxylic acid (HMFCA), and producing a purified FDCA pathway product, wherein the solvent is a multi-component solvent comprising water and a water-miscible aprotic organic solvent, such as dioxane; and the heterogeneous reduction catalyst comprises a solid support and a metal selected from the group consisting of Cu, Ni, Co, Pd, Pt, Ru, Ag, Au, Rh, Os, Ir, and any combination thereof. Further details regarding such a process may be found in WO2019014382. HMFCA may be removed from the furanic oxidation product comprising FDCA via re-crystallisation. Thus, FFCA may be removed from a furanic oxidation product comprising FDCA by hydrogenating it to HMFCA, which in turn may be removed by re-crystallisation of the hydrogenated furanic oxidation product comprising FDCA. Alternatively, hydrogenation may be performed by performed under elevated temperature (such as about 150-200 °C) and elevated pressure (such as about 5-15 bar H₂) in a water as the hydrogenation solvent and by using a Pd-C hydrogenation catalyst.

The methods of the present document may further comprise keeping a hydrogenated furanic oxidation product comprising FDCA, wherein the furanic oxidation product comprising FDCA also comprises an antioxidant, at an elevated temperature. Typically, the hydrogenated furanic oxidation product comprising an antioxidant is held at an elevated temperature for at least 0.5 hours, such as for from about 0.5 hours to about 24 hours, such as from about 10 minutes to about 12 hours.

The methods of the present document may further comprise a step of re-crystallising the hydrogenated furanic oxidation product comprising FDCA to which an antioxidant has been added. Such a re-crystallised hydrogenated furanic oxidation product typically comprises 200 ppm or less of non-FDCA furans, preferably 100 ppm of non-FDCA furans. The non-FDCA furans of such a hydrogenated re-crystallised furanic oxidation product are typically FFCA and 2-furoic acid and very small amounts of HMFCA. Typically, most or all of the antioxidant will be removed if the hydrogenated furanic oxidation product comprising FDCA is re-crystallised. A re-crystallised composition comprising a furanic oxidation product comprising FDCA therefore contains little, substantially no or no antioxidant. Re-crystallisation is a process well-known to the person skilled in the art. For example, the re-crystallisation may be performed by cooling crystallisation or evaporative crystallisation or a combined evaporative-cooling crystallisation or any other method known in the art of crystallisations. The crystallization may be conducted in batch mode or in continuous mode.

The methods of the present document may further comprise a step of oxidising a furanic oxidation substrate to produce a furanic oxidation product. The method by which the furanic oxidation product comprising FDCA is produced is not critical for the purposes of the present document and many different methods for producing a furanic oxidation product comprising FDCA are known in the art. An exemplary method for oxidising a furanic oxidation substrate to produce a furanic oxidation product is disclosed in WO2017123763. In such a method a heterogenous catalyst is used (e.g. Pt, Au or Rh on a solid support). Another exemplary method for producing the furanic oxidation product is the so-called AMOCO process, which e.g. is disclosed in WO2012161973. In such a process a homogenous catalyst, such as a Co, Mn or Br catalyst, is used. The furanic oxidation substrate for producing a furanic oxidation product may comprise hydroxymethyl furfural (HMF). The furanic oxidation substrate may in addition or alternatively comprise FFCA, HMFCA and/or DFF. Typically, the oxidation substrate comprises or consists of HMF.

Thus, a method for preventing discoloration of a furanic oxidation product comprising FDCA according to the present document may in accordance with the above comprise or consist of the following steps:
i) providing a furanic oxidation product comprising FDCA;
ii) hydrogenating the furanic oxidation product comprising FDCA of step i) to provide a hydrogenated furanic oxidation product comprising FDCA;
iii) optionally re-crystallising the hydrogenated furanic oxidation product comprising FDCA of step ii),
wherein the method further comprises a step of adding an antioxidant to the furanic oxidation product comprising FDCA of step i) before performing step ii) and/or adding an antioxidant to the hydrogenated furanic oxidation product comprising FDCA of step ii) before performing step iii). Such a method may further comprise a step of oxidising a furanic oxidation substrate, which oxidation is performed to provide the furanic oxidation product comprising FDCA of step i). The method may further comprise a step performed after step ii) but before step iii) of keeping the hydrogenated furanic oxidation product at an elevated temperature as disclosed elsewhere herein. If the antioxidant is added after hydrogenation of the furanic oxidation product comprising FDCA, it is preferably added as soon as possible after hydrogenation, in particular if the furanic oxidation product comprising FDCA is to be kept at an elevated temperature.

The present document is also directed to a method for producing a colour-stabilized furanic oxidation product comprising FDCA, wherein the method comprises the steps of
i) providing a furanic oxidation product comprising FDCA;
ii) hydrogenating the furanic oxidation product comprising FDCA of step i) to provide a hydrogenated furanic oxidation product;
iii) optionally re-crystallising the hydrogenated furanic oxidation product comprising FDCA of step ii),
wherein the method further comprises a step of adding an antioxidant to the furanic oxidation product comprising FDCA of step i) before performing step ii) and/or adding an antioxidant to the hydrogenated furanic oxidation product comprising FDCA of step ii) before performing step iii). Such a method may further comprise a step of oxidising a furanic oxidation substrate, which oxidation is performed to provide the furanic oxidation product comprising FDCA of step i). The method may further comprise a step performed after step ii) but before step iii) of keeping the hydrogenated furanic oxidation product at an elevated temperature as disclosed elsewhere herein. If the antioxidant is added after hydrogenation of the furanic oxidation product comprising FDCA, it is preferably added as soon as possible after hydrogenation, in particular if the furanic oxidation product comprising FDCA is to be kept at an elevated temperature. Details regarding the furanic oxidation product, the antioxidant, hydrogenation step, re-crystallisation step etc. are as given elsewhere herein.

The furanic oxidation product comprising FDCA produced by a method of the present document has a low degree of discoloration. Such a furanic oxidation product comprising FDCA further has a low degree of impurities, in particular impurities which may cause discoloration of the furanic oxidation product comprising FDCA and/or polymerised products produced therefrom.

The present document is also directed to a composition comprising a hydrogenated furanic oxidation product comprising FDCA and an antioxidant. Details regarding the hydrogenated furanic oxidation product comprising FDCA and the antioxidant in such a composition are as disclosed elsewhere herein.

Further, the present document is also directed to a composition comprising a hydrogenated furanic oxidation product comprising FDCA and an antioxidant obtained or obtainable by a method according to the present disclosure.

The present document is also directed to the use of an antioxidant as disclosed herein to prevent discoloration of a hydrogenated furanic oxidation product comprising FDCA. As disclosed elsewhere herein, the antioxidant may be added before and/or after hydrogenation.

The invention will be further described in the following example, which does not limit the scope of the invention described in the claims.

### EXPERIMENTAL SECTION

General experimental conditions:
Hydrogenation catalyst: Crushed or powdered catalyst from commercial suppliers, see experimental tables.

H₂-feed: FDCA solution (4.4 wt% FDCA, 0.7 wt% FFCA) in 1,4-dioxane-water from a continuous HMF oxidation process, as described in WO2019014382.

In certain control experiments, the H2-feed were pure, commercially available FDCA, FFCA and HMFCA. They were used in equal amounts (4 g) as 5 wt% solutions in 1,4-dioxane-water (70:30 wt:wt).

Antioxidant: The antioxidant was added in a 1:300 wt:wt ratio to the amount of FDCA used.

Hydrogenation reaction conditions: 136 mg of crushed or powder hydrogenation catalyst was added to a hydrogenation vial. H₂-feed (4 g) was added to the vial. The reaction vial was sealed in an Endeavour Pressure Reactor. The reaction vial was degassed with N₂ and heated to the working temperature of 130-170 °C. The reaction vial was pressured to 5-7 bar using hydrogen gas. The reaction mixture was stirred for 2 h at the desired temperature and pressure after which the temperature was decreased to 80 °C. The reaction vial was depressurized and purged with N₂ before it was filtered through a 0.45 µm PTFE filter to remove the catalyst. The vial was degassed with N₂ before addition of the antioxidant (if applicable). The vial was sealed and heated to 130 °C for 12 h. The colour of the hydrogenated and heat-treated reaction mixture was compared to the original H2-feed, with or without heat treatment at 130 °C for 12 h.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Unless expressly described to the contrary, each of the preferred features described herein can be used in combination with any and all of the other herein described preferred features.

## Claims

1. A method for preventing discoloration of a hydrogenated furanic oxidation product comprising furandicarboxylic acid (FDCA), said method comprising adding an antioxidant to a furanic oxidation product comprising FDCA, wherein said antioxidant is added before and/or after hydrogenation of said furanic oxidation product comprising FDCA.

2. The method according to claim 1, wherein said furanic oxidation product comprising FDCA further comprises 5-formyl-2-furancarboxylic acid (FFCA) and/or 5-hydroxymethyl-2-furancarboxylic acid (HMFCA).

3. The method according to any one of the preceding claims, wherein said antioxidant is added after hydrogenation of said furanic oxidation product comprising FDCA.

4. The method according to any one of the preceding claims, wherein said furanic oxidation product comprising FDCA is produced by oxidising a furanic oxidation substrate.

5. The method according to claim 4, wherein said furanic oxidation substrate is hydroxymethyl furfural (HMF), diformylfuran (DFF), hydroxymethylfurancarboxylic acid (HMFCA), and 5-formylfurancarboxylic acid (FFCA).

6. The method according to claim 4 or 5, wherein said furanic oxidation substrate comprises or consists of HMF.

7. The method according to any one of the preceding claims, wherein said antioxidant is a substituted phenol and/or a phosphine.

8. The method according to any one of the preceding claims, wherein said antioxidant is selected from one or more of ascorbate, ascorbic acid, sodium metabisulfite, butyl hydroxytoluene (BHT), tert-butylhydroquinone (TBHQ) and butylated hydroxyanisole (BHA).

9. The method according to any one of the preceding claims, said method further comprising keeping said hydrogenated furanic oxidation product comprising FDCA and an antioxidant, at an elevated temperature.

10. The method according any one of the preceding claims, said method further comprising a step of re-crystallising said hydrogenated furanic oxidation product comprising FDCA and an antioxidant.

11. A method for producing a colour-stabilized furanic oxidation product comprising FDCA, said method comprising the steps of:
i) providing a furanic oxidation product comprising FDCA;
ii) hydrogenating the furanic oxidation product comprising FDCA of step i) to provide a hydrogenated a furanic oxidation product;
iii) optionally re-crystallising the hydrogenated furanic oxidation product comprising FDCA of step ii),
said method further comprising a step of adding an antioxidant to the furanic oxidation product comprising FDCA of step i) before performing step ii) and/or adding said antioxidant to said hydrogenated furanic oxidation product comprising FDCA of step ii).

12. A composition comprising a hydrogenated a furanic oxidation product comprising FDCA and an antioxidant.

13. A composition comprising a hydrogenated furanic oxidation product comprising FDCA and an antioxidant obtained or obtainable by a method according to any one of the preceding claims.

14. Use of an antioxidant to prevent discoloration of a hydrogenated furanic oxidation product comprising FDCA.
